# EUROPEAN PATENT SPECIFICATION

(11) **EP 0 382 781 B1**
(45) Date of publication and mention of the grant of the patent: **29.09.1993**
(21) Application number: 88910316.4
(22) Date of filing: 21.10.1988
(51) Int. Cl.: A61K 37/02, A61K 31/705, C07K 17/06, C07H 15/24, A61K 47/42

(54) **COMPOSITION FOR TREATING CANCERS CHARACTERIZED BY OVER-EXPRESSION OF THE C-FMS PROTO-ONCOGENE**
ZUSAMMENSETZUNG ZUR BEHANDLUNG VON KREBS, GEKENNZEICHNET DURCH ÜBEREXPRESSION DES C-FMS-PROTO-ONKOGENS
COMPOSITION POUR LE TRAITEMENT DES CANCERS CARACTERISES PAR LA SUREXPRESSION DU PROTO-ONCOGENE C-FMS

(30) Priority: 23.10.1987 US 112801
(43) Date of publication of application: 22.08.1990
(73) Proprietor: GENETICS INSTITUTE, INC., Cambridge, Massachusetts 02140 (US)
(72) Inventor: BROWN, Eugene, L., Newton Highlands, MA 02161 (US)
(74) Representative: VOSSIUS & PARTNER
(86) International application number: US8803697
(87) International publication number: WO8903687

(56) References cited:
- EP-A- 0 329 185
- US-A- 4 504 586
- US-A- 4 675 382
- SCIENCE, vol. 236, June 1987; CLARK, pp. 1229-37#
- BLOOD, vol. 67, February 1986; METCALF, pp. 257-67#
- PHARMAC. THER., vol. 15, 1982; OLSNES, pp. 355-79#
- EXP. CLIN. CANCER RES., vol. 3, 1984; CHERSI, pp. 217-23#
- MONOCLONAL ANTIBODIES '84: Biological and Clinical Applications, 1985; THORPE, pp. 475-506#

## Description

The present invention refers generally to the treatment of a variety of cancers characterized by the over-expression of the protein receptor, c-fms. More specifically, the invention refers to a composition for such treatment including the M-CSF polypeptide linked to a cytotoxic agent.

A variety of oncogenes have been associated with specific cancers. The oncogene fms has come under recent scrutiny as being related to breast, lung pancreatic, ovarian, renal, and possibly other carcinomas, including acute myelocytic leukemia (AML). See, e.g., D. J. Slamon et al, Science, 224:256-262 (1984); C. Walker et al, Proc. Natl. Acad. Sci., USA, :1804-1808 (April 1987). See also, J. H. Ohyashiki et al, Cancer Genet. Cytogenet., 25:341-350 (1987); H. D. Preisler et al, Cancer Research, 47:874-880 (Feb. 1987); C. W. Rettenmier et al, J. Cell. Biochem., 33:109-115 (1987); and R. Sacca et al, Proc. Natl. Acad. Sci. USA, 82:3331-3335 (1986). The product of the c-fms proto-oncogene is believed to be related to, and possibly identical with, a receptor of macrophage colony-stimulating factor (M-CSF). See, e.g., C. J. Sherr et al, Cell, 41:665-676 (1985);

There remains a need in the treatment of such cancers for therapeutic products capable of destroying the carcinoma cells without severely adversely affecting the patient otherwise.

As one aspect of the invention there is provided a composition for treating cancers which are characterized by high level expression of the c-fms proto-oncogene/M-CSF receptor gene. The composition includes M-CSF polypeptide (or the active fragment thereof) crosslinked to a cytotoxic agent, which is capable of crossing the membrane of the cell bearing the c-fms gene product/M-CSF receptor and acting in the cytoplasm to destroy the cell. Preferred cytotoxic agents include A and B chain toxins, A chain toxins and genetically engineered toxins.

A further aspect of the invention involves a method for making the M-CSF/cytotoxic agent composition. The M-CSF and toxin may be linked by employing one or more heterofunctional or bifunctional protein cross linkers or by genetic fusion. The bifunctional cross-linkers are chosen to ensure that the M-CSF/toxin composition is stable while the composition is homing to the target cell. At the same time the crosslinker has to permit the release of the toxin portion after the M-CSF/toxin composition has entered the cell. See, e.g. Molecular Action of Toxins and Viruses, P. Cohen and S. van Heyningen, eds., Elsevier, New York, pp51-105 (1982).

Other aspects and advantages of the present invention will be apparent upon consideration of the following detailed description of the invention, including illustrative examples of the practice thereof.

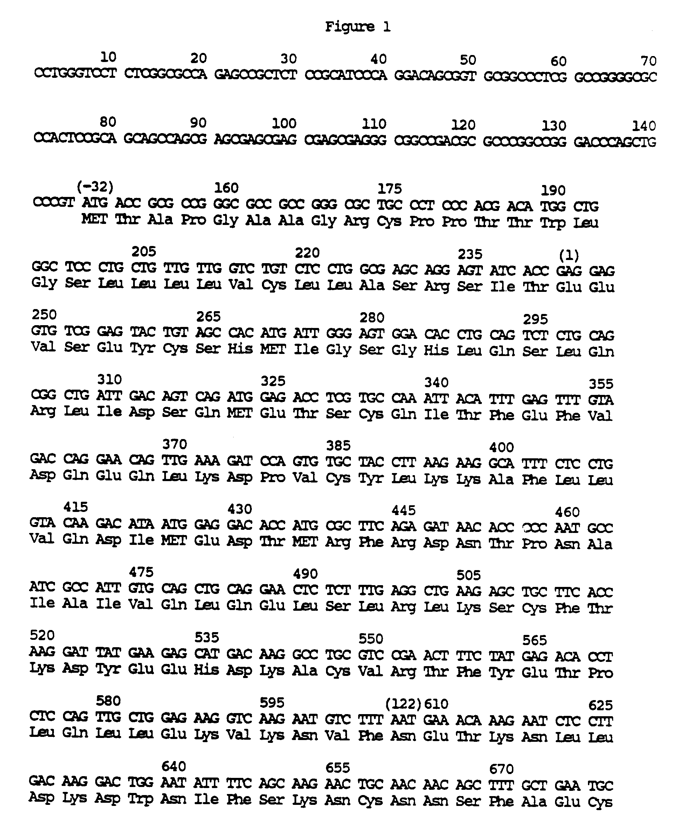

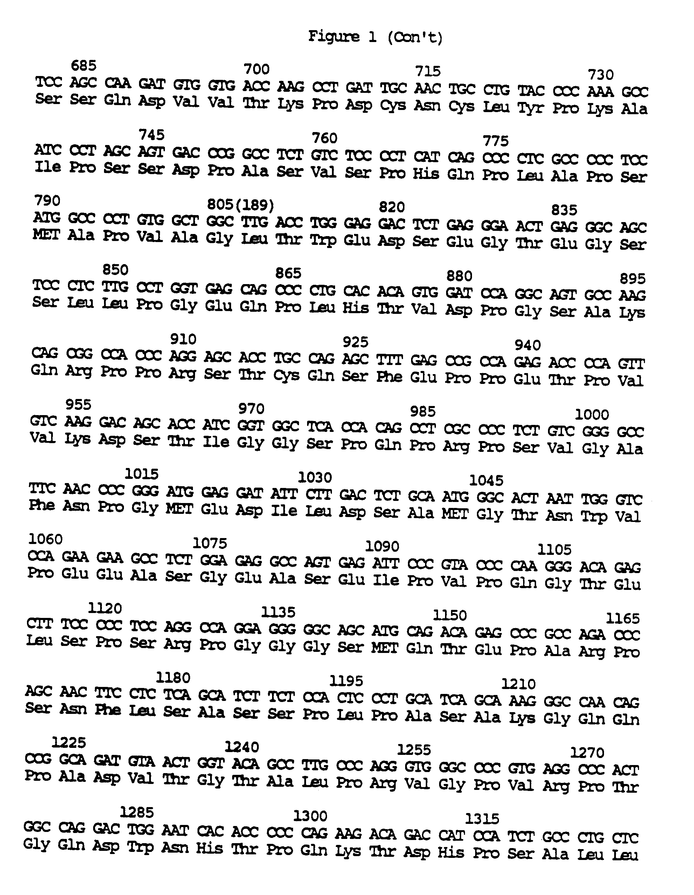

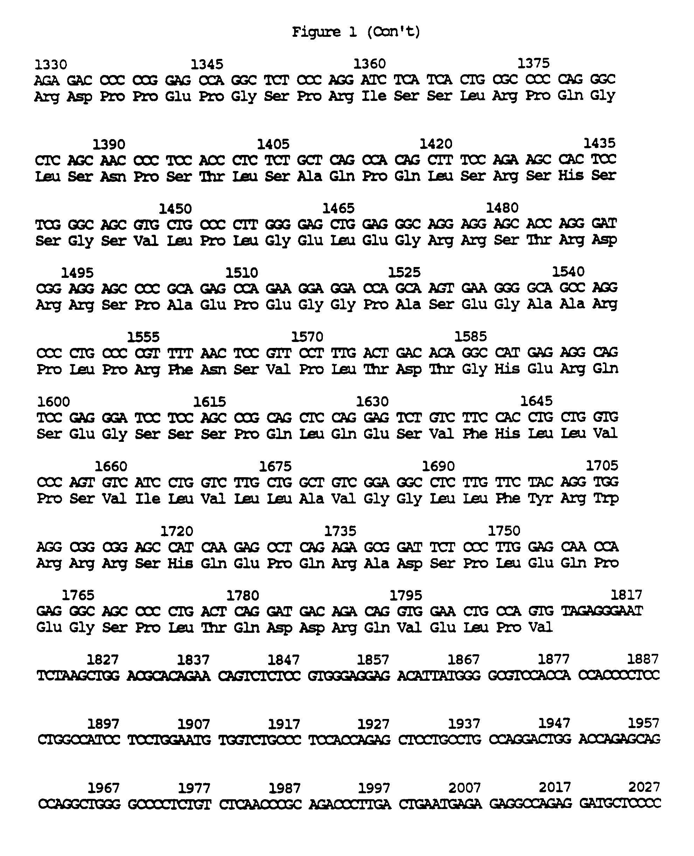

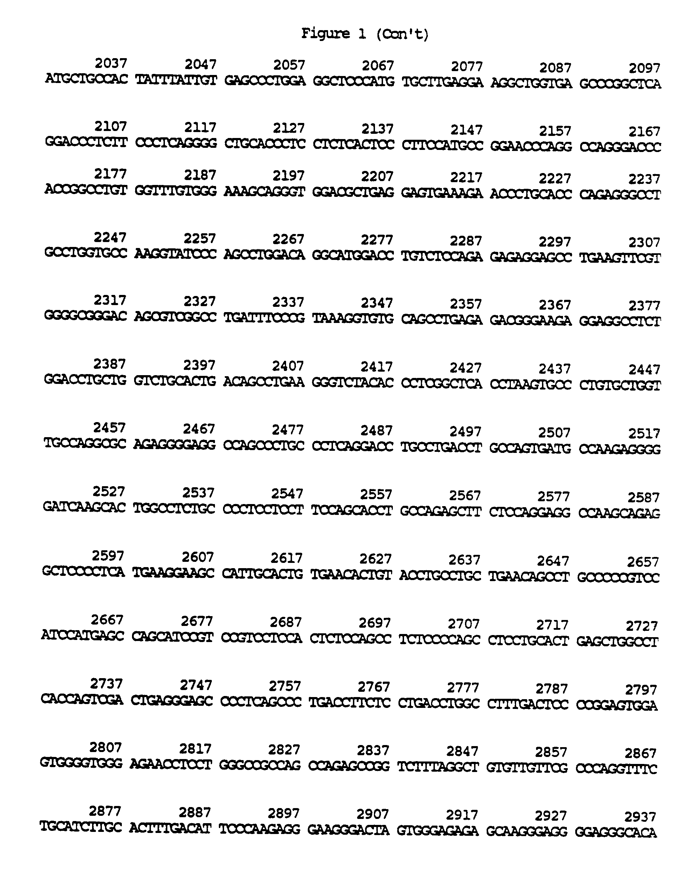

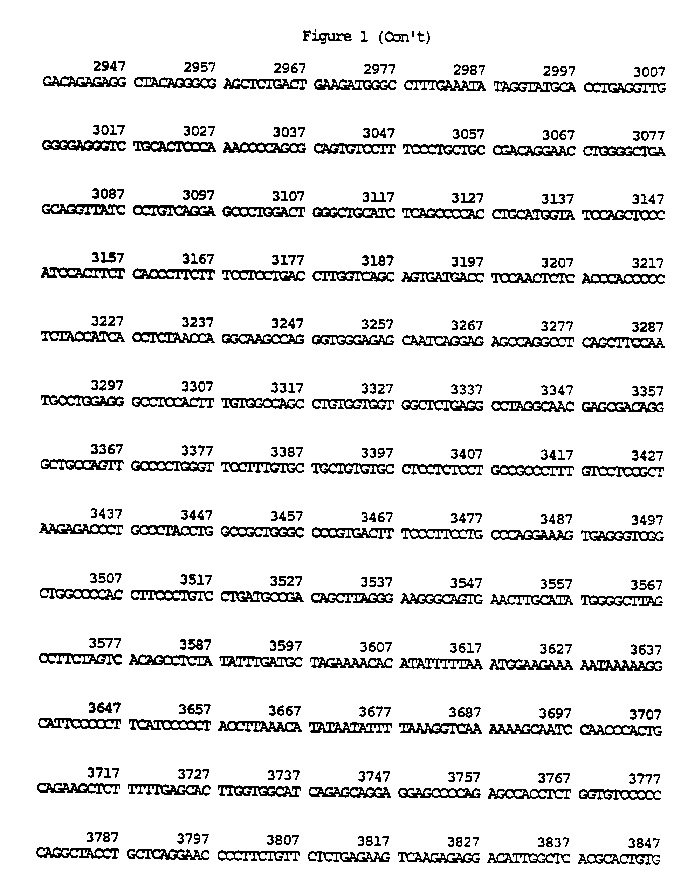

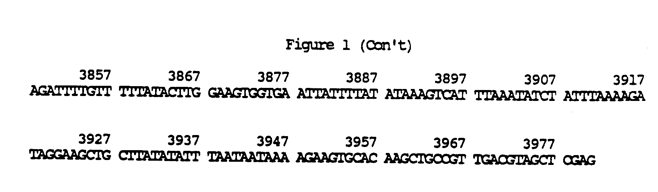

Fig. 1 illustrates a DNA and amino acid sequence for an M-CSF polypeptide.

The therapeutic composition of the invention is a conjugate of M-CSF, which is capable of binding to the c-fms proto-oncogene/M-CSF receptor gene product on certain cancer cells, and a cytotoxic agent capable of being transported through the cell membrane and acting in the cytoplasm to destroy the cell.

The M-CSF for use in the present invention may be recovered from natural sources and purified. (See e.g, UK Patent 2,016,477 and PCT published application WO86/04587). Alternatively, the M-CSF may be produced recombinantly. One possible recombinant M-CSF polypeptide useful in the present invention has been described in PCT published application WO86/04607. Another M-CSF polypeptide is described in co-pending, co-owned US patent application SN940,362 and in G. G. Wong et al, Science, 235:1504-1508 (1987). The amino acid and DNA sequence of the M-CSF described therein is presented hereto in Fig. 1. Other forms of M-CSF bearing the active site thereof may also be employed in this composition, including synthetically produced polypeptides or polypeptides modified by recombinant means.

The term "M-CSF" is herein defined as including the naturally occurring human polypeptide M-CSF and naturally-occurring allelic variations of the polypeptide. Allelic variations are naturally-occurring base changes in the species population which may or may not result in an amino acid change in a polypeptide or protein. Additionally included in this definition are both recombinant and synthetic versions of the polypeptide M-CSF, which may contain induced modifications in the peptide and DNA sequences thereof.

For example, the M-CSF polypeptide in the composition of the present invention may be characterized by a peptide sequence the same as or substantially homologous to the amino acid sequence illustrated in Fig. 1. These sequences may be encoded by the DNA sequence depicted in Fig. 1 or sequences containing allelic variations in base or amino acid sequence or deliberately modified structures coding for polypeptides with M-CSF biological properties.

Synthetic M-CSF proteins for use in the composition of the present invention may wholly or partially duplicate continuous sequences of the amino acid residues of Fig. 1. These sequences, by virtue of sharing structural and conformational characteristics with M-CSF polypeptides, e.g., the active site of the polypeptide of Fig. 1, may also possess M-CSF biological properties. Thus synthetic or recombinant polypeptides or fragments thereof may also be employed as biological or immunological equivalents for M-CSF polypeptides in the composition and methods of the present invention.

M-CSF, as used in the present invention also includes factors encoded by sequences similar to Fig. 1, but into which modifications are naturally provided or deliberately engineered. Modifications in the peptide or sequence of M-CSF can be made by one skilled in the art using known techniques. Specific modifications of interest in the M-CSF related sequences may include the replacement of one or more of the nine cysteine residues in the coding sequence with other amino acids. Preferably several cysteines in each sequence are replaced with another amino acid, e.g. serine, to eliminate the disulfide bridges at those points in the protein. For example, lysine at amino acid position 163 (Fig. 1) could be deleted or substituted with another amino acid in order to eliminate the sensitivity of this region of M-CSF to trypsin-like proteases. Mutagenic techniques for such replacement are well known to one skilled in the art. [See, e.g., United States patent 4,518,584.]

Other specific mutations of the sequence of M-CSF described herein involve modifications of one or more of the glycosylation sites in the sequence. The absence of glycosylation or only partial glycosylation results from amino acid substitution or deletion at one, two, three or all of the asparagine-linked glycosylation recognition sites present in the sequence of M-CSF. The asparagine-linked glycosylation recognition sites comprise tripeptide sequences which are specifically recognized by appropriate cellular glycosylation enzymes. These tripeptide sequences are either asparagine-X-threonine or asparagine-X-serine, where X is usually any amino acid. A variety of amino acid substitutions or deletions at one or both of the first or third amino acid positions of a glycosylation recognition site (and/or amino acid deletion at the second position) results in non-glycosylation at the modified tripeptide sequence. Modification and variation of the types of oligosaccharides which attach to the O or N-linked glycosylation sites can occur by production of the sequence in either mammalian, bacterial, yeast or insect cells. Such modifications in the proteins are also encompassed by the term M-CSF.

Yet further modifications of M-CSF polypeptides may employ sequences which are designed for improved pharmacokinetics, by, e.g., association with polyethylene glycol. Alternatively, the last 25 to 35 amino acids of the mature protein can be eliminated by appropriate gene deletion techniques to provide another form of M-CSF for use in the present invention. Such a deleted M-CSF may have use in genetic fusion to a cytotoxic agent. Amino acid residues 464 to 485 comprise a potential hydrophobic membrane-penetrating region. An M-CSF molecule that contains this sequence may desirably be employed in the composition of the invention, because these residues may embed the conjugate in the cell membrane, thereby aiding in the transfer of the cytotoxic agent into the cytosol.

An exemplary DNA sequence for the production of various M-CSF peptides have been deposited with the American Type Culture Collection, 12301 Parklawn Drive, Rockville, MD. The cDNA sequence illustrated in Fig. 1 below in vector p3ACSF-69, included in E. coli HB101 has been deposited on April 16, 1986 and given accession number ATCC 67092. This deposit was made under the Budapest Treaty on the International Recognition of the Deposit of Microorganisms for the Purposes of Patent Procedure and the Regulations thereunder (Budapest Treaty).

The cytotoxic agent linked to the M-CSF polypeptide is preferably a toxin or chemical agent which is capable of acting in the cytoplasm. Toxins may be employed which have a translocation property to move it through the cell membrane and a cytolytic domain, which provides its killing ability. One preferable class of toxins well-suited for this composition consists of two functionally different parts, termed A and B, which are connected by a disulfide bond. The A chain portion contains the enzymatic activity that enters the cytosol and kills the cell. The B chain moiety is responsible for binding of the toxin to the cell and presumably contains a domain that aids the A chain in crossing the cell membrane. Exemplary toxins for such use include native or genetically engineered ricin, abrin, modeccin, viscumin, Pseudomonas aeruginosa exotoxin, Diphtheria toxin, Cholera toxin, Shigella toxin and E. coli heat labile toxin. The toxin portion of a conjugate prepared according to the invention can consist of the cytotoxic A chain portion only, the native holotoxin, or an engineered holotoxin, i.e., a toxin lacking its lectin binding property.

Other toxins which have only a single chain (an A chain portion) may also be employed. Examples of these toxins are ribosome inactivating proteins, such as pokeweed antiviral protein and gelonin. See, L. Barbieri et al, Cancer Surveys, 1:489-520 (1982) for a more complete list of ribosome inactivating proteins.

Mutant toxins or genetically engineered toxins may also be employed. Additionally microbially produced cytotoxic agents, and other non-protein organic molecules may be used as cytotoxic agents. The M-CSF ligand can also be linked to cytotoxic drugs, such as anthracyclines, e.g., doxorubicin, daunomycin, and the vinca alkaloids, such as, vindesine, vinblastine, vincristine. Methotrexate and its derivatives may also be employed as cytotoxic agents. More effective agents are those in which many molecules (between 5 to 50) of the drug are linked to the M-CSF through a polymer carrier, e.g., dextran. Bonds linking the drug to the carrier should be cleavable by the chemical environment inside the cell.

The M-CSF and a cytotoxic agent may be linked in a variety of ways. One way of linking these components is by employing one or more standard bifunctional protein crosslinkers, such as succinimidyl 3-(2-pyridyldithio)propionate (SPDP) or succinimidyl acetylthiopropionate (SATP). These crosslinkers form stable disulfide bonds between the M-CSF and toxin, or other cytotoxic agent, and yet are capable of releasing the toxin portion of the composition inside the cell, due to cleavage of the disulfide bonds by chemicals inside the cell, e.g., intracellular glutathione. These linking methods are known to those skilled in the art. See, e.g., J. Carlsson et al, Biochem. J., 173:723-737 (1978) and N. Fujii et al, Chem, Pharm. Bull., 33:362-367 (1985). See also, A. J. Cumber et al, Methods Enzymol., 112:207-225 (1985) for other general methods for conjugating toxins to proteins.

For example, one method according to the invention involves making a M-CSF-toxin composition, using a toxin having both and A and B chain. The method involves the steps of:
(a) reacting the M-CSF with sufficient crosslinker to introduce between 1 to 6 reactive groups per molecule of M-CSF. A sufficient amount of crosslinker which can be used for this purpose is between approximately 6 to 50 moles of crosslinker per mole of M-CSF dimer.
(b) reacting a toxic protein having A and B chain subunits connected by at least one disulfide bond with a conventional reducing agent, thereby liberating the chains from each other.
(c) reacting the derivitized M-CSF of step (a) with the liberated A chain subunit of the reduced toxin; and
(d) separating from the reaction mixture conjugates comprising M-CSF linked by disulfide bonds to A chain subunits.
One exemplary growth factor/toxin conjugate is prepared by this method, modifying M-CSF with SPDP, followed by conjugation of ricin A chain toxin via a disulfide bond.

Another method for making the compositions of the present invention involves the following steps:
(a) reacting the M-CSF with sufficient crosslinker to introduce between 1 to 6 reactive groups per molecule of M-CSF;
(b) reacting the derivatized M-CSF of step (a) with a holotoxin having A and B subunits attached by at least one disulfide bond, the holotoxin being functionalized with a protein crosslinker which is preferably attached to the B subunit; and
(c) separating a conjugate formed by M-CSF becoming attached to the B subunit from free M-CSF and toxin in the reaction mixture.

Another manner of linking the components of the composition of the present invention is by a genetic fusion method. See, for example, United States Patent 4,675,382.

The compositions of the present invention containing both M-CSF and a toxin can be employed in methods for treating cancers characterized by over-expression of the c-fms proto-oncogene/M-CSF receptor gene. Among such cancers are acute myelocytic leukemia, ovarian cancer, breast cancer, lung cancer, pancreatic cancer and renal cancer. The composition of the invention operates by the targeting of the c-fms proto-oncogene by the M-CSF portion of the composition. Once attached to this receptor, the M-CSF molecule aids in transporting the cytotoxic agent through the cell membrane and into the cytosol. Inside the cell, the bonds linking the cytotoxic agent to the M-CSF are cleaved by chemicals naturally within the cell and the agent is released to kill the cancer cell.

The composition of the present invention can be administered in a variety of ways including systemically, locally or regionally. Desirably the composition is administered regionally in vivo, to the site of the carcinoma. For example, it can be administered intraperitonially, if desired, to contain its distribution to the peritoneum for use in treating a suitable cancer, e.g., ovarian cancers. Similarly for treating lung cancers, the composition could be delivered in the form of an inhalant. If desirable, the composition may be administered subcutaneously, such as bathing effected tissue after surgical removal of a tumor e.g., for breast cancers. The composition may preferably be administered intravesically for instance into the bladder. Additionally, the composition can be employed in ex vivo applications, such as "purging" of a mixture of cells removed from a patient, for patients having a systemic cancer which is not appropriate for regional application. The treatment of patients with acute myelocytic leukemia, for example, could involve removal of bone marrow cells from the body. These cells are then treated outside the body with the composition of the present invention to destroy a subset of these cells which are overexpressing the c-fms proto-oncogene. The "purged" cells are then reintroduced into the patient. The M-CSF/toxin composition of the invention can thereby serve as a purging agent to destroy the leukemic cells in the bone marrow of AML patients about to undergo autologous bone marrow transplantation. Other ex vivo purging treatments may also employ the composition of the invention.

The therapeutic composition for use in this invention may be in the form of a pyrogen-free, parenterally acceptable aqueous solution. The preparation of such a parenterally acceptable protein solution, having due regard to pH, isotonicity, stability and the like, is within the skill of the art.

The dosage regimen involved in treating the patient with the composition according to this invention will be determined by the attending physician considering various factors which modify the action of drugs, e.g. the condition, body weight, sex and diet of the patient, the severity of any infection, time of administration and other clinical factors. Additionally, the mode of administration could effect the dosage, e.g., ex vivo or in vivo. Generally, the daily regimen should be in the range of 2 to 2000 micrograms of polypeptide per kilogram of body weight.

The following examples illustrate the production of the M-CSF polypeptide and the construction of an M-CSF/toxin conjugate of the present invention.

### EXAMPLE 1

### Recombinant Production of M-CSF

To express the recombinant M-CSF polypeptide by recombinant means, the DNA encoding the polypeptide is transferred into an appropriate expression vector and introduced into selected host cells by conventional genetic engineering techniques.

Mammalian cell expression vectors for production of M-CSF, such as p3ACSF-69, may be synthesized by techniques well known to those skilled in this art. The components of the vectors, e.g. replicons, selection genes, enhancers, promoters, and the like, may be obtained from natural sources or synthesized by known procedures. See, Kaufman et al, J. Mol. Biol., 159:511-521 (1982); and Kaufman, Proc. Natl. Acad. Sci., U.S.A., 82:689-693 (1985). Suitable cells or cell lines for the expression of these recombinant M-CSF proteins may be Chinese hamster ovary cells (CHO), monkey COS-1 cells or CV-1 cells. The selection of suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U.S. Patent 4,419,446. Other exemplary mammalian host cells include particularly primate cell lines and rodent cell lines, including transformed cell lines. Normal diploid cells, cell strains derived from in vitro culture of primary tissue, as well as primary explants, are also suitable. Candidate cells need not be genotypically deficient in the selection gene so long as the selection gene is dominantly acting. For stable integration of the vector DNA, and for subsequent amplification of the integrated vector DNA, both by conventional methods, CHO cells may be employed. Other suitable mammalian cell lines include but are not limited to, HeLa, mouse L-929 cells, 3T3 lines derived from Swiss, Balb-c or NIH mice, BHK or HaK hamster cell lines.

Stable transformants are then screened for expression of the product by standard immunological or enzymatic assays. The presence of the DNA encoding the variant proteins may be detected by standard procedures such as Southern blotting. Transient expression of the DNA encoding the variants during the several days after introduction of the expression vector DNA into suitable host cells such as COS-1 monkey cells is measured without selection by activity or immunologic assay of the proteins in the culture medium. The transformation of these vectors into appropriate host cells can result in expression of the M-CSF.

Similarly, one skilled in the art could manipulate the sequence of Fig. 1 by eliminating or replacing the mammalian regulatory sequences flanking the coding sequence with bacterial sequences to create bacterial vectors for intracellular or extracellular expression of M-CSF by bacterial cells. The DNA encoding the factor may be further modified to contain different codons for bacterial expression as is known in the art. Preferably the sequence is operatively linked in-frame to a nucleotide sequence encoding a secretory leader polypeptide permitting bacterial expression, secretion and processing of the mature variant protein, also as is known in the art. The compounds expressed in bacterial host cells may then be recovered, purified, and/or characterized with respect to physicochemical, biochemical and/or clinical parameters, all by known methods. For example, the M-CSF coding sequence could be inserted into a known bacterial vector using procedures such as described in T. Taniguchi et al., Proc. Natl Acad. Sci. USA, 77:5230-5233 (1980). This exemplary bacterial vector could then be transformed into bacterial host cells and the factor expressed thereby. The various strains of E. coli (e.g., HB101, MC1061) are well-known as host cells in the field of biotechnology. Various strains of B. subtilis, Pseudomonas, other bacilli and the like may also be employed in this method. For a strategy for producing extracellular expression of such factors in bacterial cells, see, e.g. European patent application EPA 177,343.

Similar manipulations can be performed for the construction of an insect vector [See, e.g., procedures described in published European patent application 155,476] for expression in insect cells. See, e.g. Miller et al, Genetic Engineering, 8:277-298 (Plenum Press 1986) and references cited therein.

A yeast vector could also be constructed employing yeast regulatory sequences for intracellular or extracellular expression of M-CSF by yeast cells. Many strains of yeast cells known to those skilled in the art are also available as host cells for expression of the polypeptides useful in the invention. [See, e.g., procedures described in published PCT application WO 86 00639 and European patent application EP 123,289.]

### EXAMPLE 2

### An M-CSF Toxin Conjugate

For construction of an M-CSF toxin conjugate according to the invention, the growth factor M-CSF was produced in mammalian cells as described in pending U. S. patent SN940,362 and G. G. Wong et al, Science, 235 supra. M-CSF (5 mg, 55 nmoles) in 0.1M NaHCO₃ (20 ml) was reacted with a 20-fold molar excess of SPDP in ethanol. The reaction was allowed to proceed for five hours at 4 degrees Celsius to introduce approximately four to six sulfhydryl groups per molecule of M-CSF dimer. After removal of excess SPDP the derivatized growth factor was reacted with ricin A (15 mg, 500 nmoles), obtained from a commercial source, in 50mM NaH₂PO₄ p. 117.5/OIM NaCL. The disulfide bond was allowed to form overnight at 4 degrees Celsius. The resulting M-CSF-ricin A chain conjugate was separated from excess ricin A chain by gel filtration on a Sepherogel® TSK-3000 high pressure liquid chromatography column to give a mixture of conjugate and M-CSF (7.5 mg). After two passages through a column of Blue Sepharose® developed with a gradient of NaCl, as described by P. P. Knowles and P. E. Thorpe, Anal. Biochem., 160: 440-443 (1987), the conjugate (720 mg) was obtained in a form free of M-CSF and consisted mainly of a species with one ricin A chain per M-CSF dimer.

### EXAMPLE 3

### In Vitro Cytotoxicity of M-CSF Toxin Conjugate

A level of toxicity and specificity for the M-CSF/ricin A chain conjugate was determined in a standard soft agar clonogenic assay in a manner similar to that described by Strong et al, Blood, 65: 627-635 (1985) with the NIH 3T3 and NIH 3T3-c-fms cell lines. The latter line which has been described by M. F. Roussel et al, Nature, 325: 549-552 (1987), is M-CSF receptor positive. Each cell line was mixed with either oonjugate or medium without conjugate (control) in agarose and thin layered into Petri dishes. After incubation at 37°C in standard CO₂ atmosphere for a period of 14 days, the number of colonies in each dish was counted visually. The NIH 3T3-c-fms cells control dishes which did not receive the conjugate showed 103 colonies per dish while the same cells treated with conjugate at a concentration of 4 x 10⁻⁸M gave only 3 colonies. The NIH 3T3 cells, treated with conjugate and untreated control cells mixed with medium gave 76 and 78 colonies per dish, respectively.

### EXAMPLE 4

### Ex Vivo Assay of M-CSF Toxin Conjugate

The efficacy of the M-CSF/ricin A chain conjugate for ex vivo bone marrow purging is tested in a manner analogous to that described by Strong et al, supra. M1 myeloid leukemic cells (10³) which may be obtained from the American Type Culture Collection, Rockville, Maryland, (ATCC TIB 192) are added to murine bone marrow cells (10⁵) and then treated with the M-CSF/ricin A conjugate in the 10⁻⁷ - 10⁻¹²M range for approximately 4 hours at 37C. The percent survival of the leukemic cells as well as the monopotent and pluripotent bone marrow progenitor cells is determined with a standard colony formation assay, T.R. Bradley and D. Metcalf, Aust. J. Exp. Biol. Med. Sci., 44: 287 (1966) to measure the efficacy and specificity, respectively.

Numerous modifications may be made by one skilled in the art to the methods and components of the present invention in view of the disclosure herein. Such modifications are believed to be encompassed in the appended claims.

## Claims

1. A therapeutic composition for treating carcinoma characterized by over-expression of the c-fms proto-oncogene/M-CSF receptor gene, said composition comprising a M-CSF polypeptide conjugated to a cytotoxic agent and pharmaceutical carrier therefor.

2. The composition according to claim 1, wherein said cytotoxic agent is double-chain ricin, the ricin A chain, abrin, the abrin A chain, modeccin, the modeccin A chain, Pseudomonas aeruginosa exotoxin, Cholera toxin, Shigella toxin, E. coli heat labile toxin or Diphtheria toxin, a mutant toxin thereof or a recombinant version thereof.

3. The composition according to claim 1 wherein said cytotoxic agent is a ribosome-inactivating protein, pokeweed antiviral protein or gelonin, a mutant toxin thereof or a recombinant version thereof.

4. The composition according to claim 1 wherein said cytotoxic agent is an anthracycline, doxorubicin, daunomycin, a vinca alkaloid, vindesine, vinblastine, vincristine, methotrexate or a derivative thereof.

5. The composition according to anyone of claims 1 to 4 wherein said M-CSF polypeptide is conjugated to said cytotoxic agent by a heterofunctional protein cross linking agent.

6. The composition according to claim 5 wherein said cross linking agent is succinimidyl 3-(2-pyridyl-dithio)propionate or succinimidyl acetylthiopropionate.

7. The composition according to claim 1 comprising M-CSF conjugated through SPDP to a full ricin molecule.

8. The composition according to anyone of claims 1 to 7 for the regional administration to the site of said cancer.

9. The composition according to anyone of claims 1 to 7 for in vitro purging of a mixture of cells removed from a patient, said mixture containig said cancer cells, with said M-CSF linked to said cytotoxic agent.

10. The composition according to claim 8 or 9, wherein said M-CSF linked to said cytotoxic agent is capable of crossing the membrane of the cells bearing said receptor and of entering and killing the cancer cells.

## Patentansprüche

1. Therapeutisches Mittel zur Behandlung eines Carcinoms, das durch die Überexpression des c-fms-Protooncogens/M-CSF-Rezeptorgens gekennzeichnet ist, wobei das Mittel ein mit einem cytotoxischen Mittel konjugiertes M-CSF-Polypeptid und einen pharmazeutischen Träger dafür umfaßt.

2. Mittel nach Anspruch 1, wobei das cytotoxische Mittel doppelkettiges Ricin, die Ricin A-Kette, Abrin, die Abrin A-Kette, Modeccin, die Modeccin A-Kette, Pseudomonas aeruginosa-Exotoxin, Choleratoxin, Shigella-Toxin, hitzelabiles Toxin von E. coli oder Diphtherietoxin, eine Toxinmutante davon oder eine rekombinante Version davon ist.

3. Mittel nach Anspruch 1, wobei das cytotoxische Mittel ein Ribosomen-inaktivierendes Protein, ein antivirales Protein von Phytolacca americana oder Gelonin, eine Toxinmutante davon oder eine rekombinante Version davon ist.

4. Mittel nach Anspruch 1, wobei das cytotoxische Mittel ein Anthracyclin, Doxorubicin, Daunomycin, ein Vincaalkaloid, Vindesin, Vinblastin, Vincristin, Methotrexat oder ein Derivat davon ist.

5. Mittel nach einem der Ansprüche 1 bis 4, wobei das M-CSF-Polypeptid mit dem cytotoxischen Mittel durch ein heterofunktionelles Protein-Vernetzungsmittel konjugiert ist.

6. Mittel nach Anspruch 5, wobei das Vernetzungsmittel Succinimidyl-3-(2-pyridyldithio)propionat oder Succinimidyl-acetylthiopropionat ist.

7. Mittel nach Anspruch 1, umfassend M-CSF, das über SPDP mit einem vollständigen Ricinmolekül konjugiert ist.

8. Mittel nach einem der Ansprüche 1 bis 7 für die regionale Verabreichung an der Stelle des Krebses.

9. Mittel nach einem der Ansprüche 1 bis 7 für die in vitro-Reinigung eines Gemisches von einem Patienten entnommenen Zellen, wobei das Gemisch die Krebszellen enthält, mit Hilfe des an das cytotoxische Mittel geknüpften M-CSF.

10. Mittel nach Anspruch 8 oder 9, wobei das an das cytotoxische Mittel geknüpfte M-CSF zur Überwindung der Membran der diesen Rezeptor tragenden Zellen und zum Eindringen und Abtöten der Krebszellen fähig ist.

## Revendications

1. Composition thérapeutique destinée au traitement de carcinomes caractérisés par la surexpression du gène de proto-oncogène c-fms/récepteur de M-CSF, composition qui comprend un polypeptide M-CSF conjugué à un agent cytotoxique et un véhicule ou excipient pharmaceutique convenable.

2. Composition suivant la revendication 1, caractérisée en ce que l'agent cytotoxique est la ricine à double chaîne, la chaîne de ricine A, l'abrine, la chaîne d'abrine A, la modeccine, la chaîne de modeccine A, l'exotoxine de Pseudomonas aeruginosa, la toxine du choléra, la toxine de Shigella, la toxine thermolabile d'E. coli ou la toxine de diphtéria, une toxine mutante de celle-ci ou une version recombinante de celle-ci.

3. Composition suivant la revendication 1, caractérisée en ce que l'agent cytotoxique est une protéine inactivant les ribosomes, la gélonine ou la protéine antivirale de phytolacca, une toxine mutante de celle-ci ou une version recombinante de celle-ci.

4. Composition suivant la revendication 1, caractérisée en ce que l'agent cytotoxique est une anthracycline, la doxorubicine, la daunomycine, un alcaloïde de vinca, la vindésine, la vinblastine, la vincristine, le méthotrexate ou un dérivé de ces composés.

5. Composition suivant l'une quelconque des revendications 1 à 4, caractérisée en ce que le polypeptide M-CSF est conjugué à l'agent cytotoxique précité par un agent de réticulation de protéines hetérofonctionnelles.

6. Composition suivant la revendication 5, caractérisée en ce que l'agent de réticulation précité est le 3-(2-pyridyldithio)propionate de succinimidyle ou l'acétylthiopropionate de succinimidyle.

7. Composition suivant la revendication 1, comprenant du M-CSF conjugué par l'intermédiaire de SPDP à une molécule de ricine entière.

8. Composition suivant l'une quelconque des revendications 1 à 7, destinée à l'administration régionale au site du cancer précité.

9. Composition suivant l'une quelconque des revendications 1 à 7, destinée à la purge in vitro d'un mélange de cellules prélevées d'un patient, le mélange en question contenant les cellules cancéreuses précitées, avec le M-CSF susmentionné lié audit agent cytotoxique.

10. Composition suivant la revendication 8 ou 9, caractérisée en ce que le M-CSF lié audit agent cytotoxique est capable de traverser la membrane des cellules porteuses du récepteur susmentionné et de pénétrer dans les cellules cancéreuses et de tuer celles-ci.
